## Europäisches Patentamt
## European Patent Office
## Office européen des brevets

(11) Veröffentlichungsnummer: **0 563 584 B1**

# EUROPÄISCHE PATENTSCHRIFT

(45) Veröffentlichungstag der Patentschrift: **27.09.95**

(51) Int. Cl.6: **C07C 409/24**, C07C 409/26, C07C 407/00, C07C 409/00

(21) Anmeldenummer: **93103178.5**

(22) Anmeldetag: **27.02.93**

(54) **Stabilisierte Percarbonsäurelösungen und Verfahren zu deren Herstellung.**

(30) Priorität: **30.03.92 DE 4210425**

(43) Veröffentlichungstag der Anmeldung:
**06.10.93 Patentblatt 93/40**

(45) Bekanntmachung des Hinweises auf die
Patenterteilung:
**27.09.95 Patentblatt 95/39**

(84) Benannte Vertragsstaaten:
**BE DE ES FR GB IT NL**

(56) Entgegenhaltungen:
**FR-A- 2 076 067**

(73) Patentinhaber: **Degussa Aktiengesellschaft**
**Weissfrauenstrasse 9**
**D-60311 Frankturt (DE)**

(72) Erfinder: **Bewersdorf, Martin, Dr.**
**Vorm Hain 4**
**W-6458 Rodenbach (DE)**
Erfinder: **Goor, Gustaaf, Dr.**
**Gleiwitzer Strasse 1**
**W-6450 Hanau 9 (DE)**

**Beschreibung**

Die Erfindung betrifft stabilisierte Percarbonsäurelösungen, welche den SADT-Test "mindestens 60 °C" erfüllen, insbesondere solche, welche im wesentlichen eine wasserlösliche Percarbonsäure, die ihr zugrundeliegende Carbonsäure, Wasserstoffperoxid, Wasser, Mineralsäure und mindestens einen Stabilisator enthalten. Ein weiterer Gegenstand richtet sich auf ein Verfahren zur Herstellung der stabilisierten Percarbonsäurelösungen.

Percarbonsäurelösungen, insbesondere solche wasserlöslicher niederer Percarbonsäuren, etwa der Peressigsäure und Perpropionsäure, finden vielseitige Anwendung. Wasserhaltige Percarbonsäurelösungen finden unter anderem Einsatz in Wasch-, Bleich- und Reinigungsmitteln sowie in mikrobiozid wirksamen Zusammensetzungen für Desinfektionszwecke im industriellen aber auch häuslichen Bereich. Percarbonsäurelösungen in wasserarmen oder wasserfreien organischen Lösungsmitteln werden als Oxidationsmittel in der chemischen Synthese eingesetzt.

Die niederen Percarbonsäuren sind aufgrund ihrer Brand- und Explosionsgefahr äußerst problematisch bezüglich ihrer Herstellung und Handhabung. In der Praxis werden daher nur Lösungen von Percarbonsäuren hergestellt und verwendet, wobei das Lösungsmittel bzw. Lösungsmittelgemisch organischer oder organisch-wäßriger Natur sein kann. Percarbonsäuren zersetzen sich leicht, insbesondere in Anwesenheit katalytisch wirksamer Schwermetallionen, bei erhöhter Temperatur oder erhöhtem pH-Wert. Da stabilitätsbeeinträchtigende Faktoren bei der Herstellung, Lagerung und Handhabung von Percarbonsäurelösungen in der Praxis nie ausgeschlossen werden können, werden bei und/oder nach deren Herstellung Stabilisatoren zugesetzt.

Ein aus der US-PS 2,609,391 bekannter Stabilisator für wäßrige Peressigsäurelösungen ist die Dipicolinsäure. Rohe Peressigsäurelösungen, wobei das Lösungsmittel ausgewählt ist aus der Reihe Essigsäure, Essigsäurealkylester, Aceton und bzw. oder Wasser oder deren Gemischen, lassen sich gemäß DE-PS 11 70 927 besser durch einen Zusatz von Picolinsäure stabilisieren.

Die Stabilisierung wäßriger Percarbonsäurelösungen mittels Pyrophosphat oder Pyrophosphorsäure lehrt die US-PS 2,347,434 und mittels eines polymeren Phosphats, etwa Natriumhexametaphosphat oder Natriumtripolyphosphat, die US-PS 2,590,856.

Stabilisatoren für Peroxidverbindungen, darunter Peressigsäure, können gemäß der DE-AS 11 07 207 1-Hydroxyalkyliden-1,1-diphosphonsäure und gemäß der DE-AS 15 19 484 Aminotri-(alkylidenphosphonsäuren) sein. Zur Stabilisierung von Peressigsäure- bzw. Perpropionsäurekonzentraten, enthaltend 0,5 bis 20 Gew.-% Percarbonsäure, Carbonsäure, Wasser und 25 bis 40 Gew.-% Wasserstoffperoxid werden gemäß der DE-PS 25 36 618 0,25 bis 10 Gew.-% Komplexbildner auf der Basis unterschiedlicher Phosphonsäuren eingesetzt, um eine für die Praxis ausreichende Lagerstabilität zu erzielen.

Um die Stabilität von Percarbonsäurelösungen zu erhöhen und/oder die Gesamtmenge an Stabilisatoren zu begrenzen, werden auch unterschiedliche Stabilisatorkombinationen verwendet. Beispielsweise lehrt die DE-AS 12 80 239 eine Kombination aus Chinolin und einem kondensierten Phosphat, die DE-OS 24 51 904 eine Kombination aus Dipicolinsäure und Natriumhexametaphosphat.

Schließlich ist in der EP 0 421 974 A1 für verdickte Peressigsäurelösungen eine Kombination aus Chelatkomplexbildnern und Radikalfängern vorgeschlagen worden.

Eine synergistisch wirksame Stabilisatorkombination für Peressigsäurelösungen, welche Peressigsäure, Essigsäure, Wasserstoffperoxid, Wasser und Mineralsäure enthalten, besteht gemäß der WO 91/07375 aus Dipicolinsäure und mindestens einer mit zweiwertigen Metallen zur Komplexbindung befähigten Phosphonsäure. Vorzugsweise enthalten derartige, durch Gleichgewichtseinstellung eines Gemischs aus Essigsäure, $H_2O_2$ und Wasser in Gegenwart einer Mineralsäure erhaltene Peressigsäurelösungen 0,1 bis 20 Gew.-% Phosphonsäure, wie z. B. 1-Hydroxyethan-1,1-diphosphonsäure, und 0,1 bis 5000 mg/l Dipicolinsäure. Auch gemäß der WO 91/13058 wird für Gleichgewichtsperessigsäure beispielgemäß eine gleichartige Kombination aus 1 % Dipicolinsäure und 1 % 1-Hydroxyethylidendiphosphat eingesetzt. Die Gesamtmenge an Stabilisatoren ist im Falle ausreichender Stabilisierung trotz der synergistischen Wirksamkeit der beiden Stabilisatorkomponenten sehr hoch und für manche Einsatzgebiete hinderlich.

Wäßrige Wasserstoffperoxidlösungen werden in stabilisierter Form in den Handel gebracht. Zur Stabilisierung werden dabei Stannate, häufiger jedoch eine Kombination aus einem Stannat, einer Phosphonsäure oder deren Salzen und/oder einem Pyrophosphat sowie ggf. weiteren Stabilisatoren eingesetzt - siehe beispielsweise die US-Patentschriften 2,872,293 , 3,383,174 , 3,387,939 sowie die DE-OS 21 31 644. Handelsübliche wäßrige $H_2O_2$-Lösungen mit einem $H_2O_2$-Gehalt von 30 bis 70 Gew.-% enthalten Stannate üblicherweise in einer Menge von weniger als 25 mg/l berechnet als $Na_2SnO_3 \bullet 3H_2O$.

Wie die Anmelderin der vorliegenden Erfindung feststellte, ist die Stabilisierung von unter Verwendung handelsüblicher, stannatstabilisierter Wasserstoffperoxidlösungen hergestellter Gleichgewichtsperessigsäure

2

völlig unzureichend; derartige Peressigsäurelösungen weisen eine hohe Zersetzungsrate auf und erfüllen bei weitem nicht die für einen Transport vom Gesetzgeber geforderten Stabilitäts- und Sicherheitsdaten.

Aus dem Derwent Abstract 83-35915 K (15) (= JP 58 038800 A) sind flüssige Waschmittel bekannt, welche waschaktive Stoffe, ein Aktivsauerstoff-Bleichmittel und Zusatzstoffe enthalten. Unter den Bleichmitteln werden Wasserstoffperoxid, Natriumperborat, Natriumpercarbonat und Harnstoffperoxid hervorgehoben; unter den Zusatzstoffen werden unter anderem Stannate und Phosphonate genannt, wobei letztere bevorzugt werden. Hinweise zur Stabilisierung von Percarbonsäurelösungen lassen sich diesem Dokument nicht entnehmen.

Ätzlösungen für Photoresiste, welche im wesentlichen aus Permonoschwefelsäure, Schwefelsäure, Wasserstoffperoxid, Wasser und Stabilisatoren bestehen, enthalten zum Zwecke einer verlängerten Lagerfähigkeit eine lösliche Zinnverbindung und einen Phosphonat-Chelatkomplexbildner. Die Lösungen enthalten 0,01 bis 1 ppm lösliche Zinnverbindung, berechnet als Sn, und 0,05 bis 1 Gew.-% Phosphonat. Dieses Dokument richtet sich nicht auf Percarbonsäurelösungen.

Aufgabe der vorliegenden Erfindung ist die Bereitstellung von stabilisierten Percarbonsäurelösungen und insbesondere die Bereitstellung sogenannter Gleichgewichtspercarbonsäurelösungen, welche derart stabilisiert sind, daß sie mit einer möglichst geringen Gesamtkonzentration an Stabilisatoren den nach den UN-Richtlinien durchgeführten SADT-Test (self accelerated decomposition temperature) von "mindestens 60 °C" erfüllen.

Gefunden wurden stabilisierte Percarbonsäurelösungen, welche den SADT-Test "mindestens 60 °C" erfüllen, enthaltend eine Percarbonsäure, ein oder mehrere Lösungsmittel und mindestens einen Stabilisator, welche dadurch gekennzeichnet sind, daß sie als Stabilisator eine Zinnverbindung in einer Menge von mindestens 20 ppm (part per million), berechnet als Sn, enthalten und nach dem beanspruchten Verfahren erhältlich sind.

Bevorzugte Percarbonsäurelösungen enthalten eine wasserlösliche Percarbonsäure, insbesondere Peressigsäure oder Perpropionsäure.

Die Lösungsmittel beziehungsweise Lösungsmittelgemische können organischer, organisch-wäßriger oder überwiegend wäßriger Natur sein. Unter den organischen Lösungsmitteln sind die den Percarbonsäuren zugrundeliegenden Carbonsäuren besonders hervorzuheben.

Für die Praxis sind Lösungen von Bedeutung, und zwar als transportfähige Handelsware als auch als Reaktionslösung zur Herstellung von im wesentlichen organischen Percarbonsäurelösungen, welche außer der Percarbonsäure die ihr zugrundeliegende Carbonsäure und Wasser sowie zusätzlich Wasserstoffperoxid und eine Mineralsäure, vorzugsweise Schwefelsäure, enthalten. Besonders bevorzugte Lösungen sind die sogenannten Gleichgewichtspercarbonsäurelösungen, etwa Gleichgewichtsperessigsäure mit einem Gehalt von

2,5 bis 40 Gew.-% Peressigsäure,
5 bis 74 Gew.-% Essigsäure,
1 bis 30 Gew.-% Wasserstoffperoxid,
10 bis 60 Gew.-% Wasser,
0,01 bis 1 Gew.-% Mineralsäure
und vorzugsweise
4 bis 20 Gew.-% Peressigsäure,
5 bis 74 Gew.-% Essigsäure,
1 bis 30 Gew.-% Wasserstoffperoxid,
15 bis 60 Gew.-% Wasser,
0,01 bis 1 Gew.-% Mineralsäure.

Im wesentlichen Organische Percarbonsäurelösungen enthalten als Lösungsmittel außer der zugrundeliegenden Carbonsäure beispielsweise Alkylester niederer Carbonsäuren, aromatische Kohlenwasserstoffe, halogenierte Kohlenwasserstoffe oder Phosphorsäureester. Derartige Lösungen werden im allgemeinen unmittelbar nach ihrer Herstellung weiterverarbeitet, eine erfindungsgemäße Stabilisierung vermag aber deren Handhabung zu erleichtern und die Zersetzungsrate bei deren Herstellung zu vermindern.

Es wurde gefunden, daß im Gegensatz zu vielen anderen Stabilisatoren die Zinnverbindungen auch in geringer Konzentration effektiv stabilisierend wirken, sofern der Mindestwert von 20 ppm, berechnet als Sn, erreicht ist und die Stabilisatorzugabe unmittelbar vor der Umsetzung einem Reaktionspartner oder/und während und/oder nach der Umsetzung dem Reaktionsgemisch zugesetzt wurde. Sofern der Zinnstabilisator in der genannten Menge bereits längere Zeit vor der Umsetzung dem $H_2O_2$ zugesetzt wurde, wird der geforderte SADT-Wert nicht erreicht. Im Prinzip können organische Zinnstabilisatoren, wie Alkylzinnhydroxide der Formel $R_nSn(OR')_{4-n}$ mit $n = 1, 2, 3$ oder $4$ und $R = C_1$ bis $C_4$-Alkyl und $R' = H$ oder Alkyl, als auch anorganische Zinnverbindungen eingesetzt werden. Während für die im wesentlichen organischen

EP 0 563 584 B1

Percarbonsäurelösungen organische Zinnverbindungen zweckmäßig sind, werden zur Stabilisierung der bevorzugten wasserhaltigen Lösungen im allgemeinen anorganische Zinnverbindungen bevorzugt. Es handelt sich hierbei um anorganische Sn(II)- und vorzugsweise Sn(IV)-verbindungen, wobei wasserlösliche Stannate vom Typ $Me_2SnO_3 \cdot 3H_2O$, worin Me ein Alkalimetall oder Ammonium bedeutet, besonders bevorzugt werden. Einfache Zinnsalze, wie z. B. $SnCl_4$, werden in den zu stabilisierenden Lösungen im allgemeinen zu Stannaten hydrolysiert.

Die Zinnverbindungen, insbesondere anorganische Zinnverbindungen, wie der Stannate und insbesondere $Na_2SnO_3 \cdot 3H_2O$, werden zweckmäßigerweise in einer Menge von 20 bis 200 ppm, berechnet als Sn, vorzugsweise aber in einer Menge von 20 bis 50 ppm, berechnet als Sn, eingesetzt.

Die erfindungsgemäßen Percarbonsäurelösungen können außer dem/den Zinnstabilisatoren zusätzlich Stabilisatoren anderer bekannter Stoffklassen enthalten. Zu nennen sind beispielsweise Stabilisatoren aus der Reihe der (a) Chelatkomplexbildner auf der Basis von Phosphonsäuren, insbesondere Hydroxy- und Aminophosphonsäuren, Hydroxy- und Aminocarbonsäuren, N-heterocyclischen Carbonsäuren sowie Salzen, insbesondere Alkali- und Ammoniumsalzen, dieser Säuren, (b) Pyro- und Polyphosphorsäuren und deren wasserlöslichen Salzen und (c) Radikalfänger auf der Basis aklylierter Hydroxyaromaten. Hydroxy- und Aminophosphonsäuren werden als Costabilisatoren bevorzugt.

Unter den mit zweiwertigen Metallen Chelatkomplexe bildenden Phosphonsäuren sind beispielhaft zu nennen: Phosphonobernsteinsäure, 2-Phosphonobutan-1,2,4-tricarbonsäure; unter den Hydroxyphosphonsäuren 1-Hydroxyäthan-1,1-diphosphonsäure und Homologe mit 3 bis 6 C-Atomen; unter den Aminophosphonsäuren Aminotrimethylenphosphonsäure, Dimethylaminomethandiphosphonsäure, 1-Amino-1-phenylmethandiphosphonsäure, Aminoessigsäure-N,N-di(methylenphosphonsäure), Ethylendiamin-tetra-(methylenphosphonsäure), Hexamethylendiamin-tetra(methylenphosphonsäure), Diethylentriamin-penta-(methylenphosphonsäure), 3-Aminopropan-1-hydroxy-1,1-diphosphonsäure.

Unter den Chelatkomplexbildnern auf der Basis von Aminocarbonsäuren sind beispielhaft zu nennen Aminotriessigsäure, Ethylendiamintetraessigsäure, unter den Hydroxycarbonsäuren Zitronensäure und Poly-alpha-hydroxyacrylsäure, unter den N-heterocyclischen Carbonsäuren Picolinsäure und Dipicolinsäure. Unter den Radikalfängern wird beispielhaft auf 3,5 und 2,6-Bis-tert.-butyl-4-methylphenol verwiesen.

Die Einsatzmenge an Costabilisatoren wird sich nach dem gewünschten Stabilisierungsgrad und dem Verwendungszweck der Percarbonsäurelösung richten. Im Falle der bevorzugten Hydroxy- und Aminophosphonsäuren und deren Salzen hat sich eine Menge zwischen 20 und 1000 ppm, vorzugsweise zwischen 20 und 200 ppm, als zweckmäßig erwiesen. Der Vorteil der Chelatkomplexe bildenden Phosphonsäuren bzw. Phosphonate liegt neben der höheren Stabilisierung auch darin, daß es mit ihrer Hilfe möglich ist, stabilisierte Percarbonsäurelösungen herzustellen, welche auch bei langer Lagerung völlig klar bleiben.

Verfahren zur Herstellung von Percarbonsäurelösungen sind dem Fachmann bekannt. Im allgemeinen wird die Carbonsäure mit wäßrigem Wasserstoffperoxid in Gegenwart einer katalytisch wirksamen starken Säure, meist Schwefelsäure oder Phosphorsäure, aber auch stark sauren fluorierten Ionenaustauschern, umgesetzt - siehe beispielsweise DE-PS 21 25 160 und DE-PS 20 18 713. Zweckmäßigerweise sind bereits während der Umsetzung Stabilisatoren zugegen, um den Aktivsauerstoffverlust gering zu halten - siehe beispielsweise WO 91/07375 und WO 91/13058. Sofern im wesentlichen organische Percarbonsäurelösung hergestellt werden sollen, werden diese vorzugsweise durch Extraktion mittels eines organischen Lösungsmittels aus den wie oben erhaltenen wäßrigen Percarbonsäurelösungen erhalten - siehe beispielsweise DE-PS 22 62 970 und DE-PS 25 19 300.

Das Verfahren zur Herstellung von Percarbonsäurelösungen, welche den SADT-Test "mindestens 60 °C" erfüllen, durch Umsetzung der Carbonsäure mit wäßrigem Wasserstoffperoxid in Gegenwart saurer Katalysatoren und, sofern erwünscht, Überführung der im Reaktionsgemisch enthaltenden Percarbonsäure in andere Lösungsmittel ist dadurch gekennzeichnet, daß man mindestens eine Zinnverbindung in einer Menge von mindestens 20 ppm, berechnet als Sn und bezogen auf die Percarbonsäurelösung, unmittelbar vor der Umsetzung einem Reaktionspartner oder während und/oder nach der Umsetzung dem Reaktionsgemisch als Stabilisator zusetzt. Die Zinnstabilisatoren, vorzugsweise anorganische Sn-verbindungen, werden bevorzugt dem Reaktionsgemisch während, üblicherweise zu Beginn, und/oder nach der Umsetzung als solche, oder als wäßrige Lösung im Gemisch mit anderen Stabilisatoren, wie insbesondere Phosphonsäuren oder Phosphonaten, zugegeben. Ein Teil der Sn-Stabilisatoren wird im Falle der Verwendung von handelsüblichem technischen wäßrigen Wasserstoffperoxid zusammen mit diesem Reaktionspartner in das Reaktionsgemisch eingebracht werden; wie die Vergleichsbeispiele zeigen, gelangt man selbst dann nicht zu der gewünschten Stabilität der Percarbonsäurelösung, wenn eine hohe Stabilisatormenge längere Zeit vor der Umsetzung dem Wasserstoffperoxid zugefügt wird.

Zur Herstellung völlig klarer stabilisierter Percarbonsäurelösungen, welche außer der Percarbonsäure, die zugrundeliegende Carbonsäure, Wasserstoffperoxid und Wasser sowie eine Mineralsäure enthalten, ist

4

es zweckmäßig, zunächst aus einer anorganischen Zinnverbindung, etwa einem Alkalistannat, und mindestens einem Chelatkomplexbildner aus der Reihe der Hydroxy- oder Aminophosphonsäuren oder deren Salzen eine klare wäßrige Stabilisatorlösung herzustellen und diese Lösung einem Reaktionspartner oder dem Reaktionsgemisch zu Beginn, während und/oder nach der Umsetzung zuzusetzen. Wird demgegenüber zu einer mit einem Stannat stabilisierten Percarbonsäurelösung, welche je nach dem Gehalt an Zinnverbindung opaleszierend oder leicht trüb sein kann, eine Phosphonsäure oder ein Phosphonat zugefügt, so kommt es zu keiner Aufklarung der Lösung.

Die erfindungsgemäßen Percarbonsäurelösungen sind in hohem Maße trotz der geringen Stabilisatormenge sehr lagerstail. Die sogenannten Gleichgewichtspersäurelösungen mit einem Peressigsäuregehalt im Bereich von 0,5 bis 40 Gew.-% erfüllen den SADT-Test "mindestens 60 °C" bei erfindungsgemäßer Stabilisierung selbst dann, wenn nur ein Bruchteil der bei bisher eingesetzten Stabilisatoren erforderlichen Menge zugegen war. Da Stannate, wie insbesondere das Natriumstannattrihydrat, darüber hinaus billiger sind als die häufig eingesetzte Dipicolinsäure, resultiert ein weiterer Vorteil.

**Beispiele**

Herstellung und Stabilisierung 5 gew.-%iger Gleichgewichtsperessigsäure

Es werden pro Ansatz 398,5 g voll entsalztes Wasser vorgelegt. Hierzu werden unter Rühren zunächst 470,0 g $H_2O_2$ 50 gew.-%ig, anschließend 120,0 g Essigsäure und 10,0 g $H_2SO_4$ gegeben. Jeder Ansatz wird dann mit den in der Tabelle angegebenen Mengen Stabilisatoren stabilisiert. Die Proben werden 3 Tage stehen gelassen, bis sich das Gleichgewicht eingestellt hat. Alle erfindungsgemäß stabilisierten Proben bestehen die adiabatische Warmlagerung, so daß die Angabe "SADT mind. 60 °C" zulässig ist (UN-Richtlinien zu UN-Klasse 5.2; siehe Orange Book / Transport of dangerous Goods, Tests and criteria, Part II, Section 4, 1990, pages 205-209).

| Beispiel Nr. | Stabilisator (Art und Menge) | SADT mind. 60 °C |
|---|---|---|
| Vergleichsbeispiel 1 | – | nicht erfüllt |
| " 2 | 500 ppm DPS | erfüllt |

| Beispiel 1 | | 50 ppm NaSn | erfüllt |
|---|---|---|---|
| " | 2 | 50 ppm NaSn | |
| | | 30 ppm HEDP | erfüllt |
| " | 3 | 50 ppm NaSn | |
| | | 250 ppm HEDP | erfüllt |
| " | 4 | 40 ppm NaSn | erfüllt |
| " | 5 | 50 ppm NaSn | |
| | | 250 ppm DPS | erfüllt |

DPS = Dipicolinsäure

HEDP = 1-Hydroxyethan-1,1-diphosphonsäure

NaSn = $Na_2SnO_3 \cdot 3H_2O$

Über die bei der Herstellung sämtlicher Peressigsäurelösungen der Beispiele und Vergleichsbeispiele eingesetzte Wasserstoffperoxidlösung wurden etwa 5 ppm $Na_2SnO_3 \cdot 3H_2O$ und etwa 3 ppm HEDP in die Peressigsäurelösung eingebracht. Vergleichsbeispiel 1 zeigt, daß die so eingebrachte Menge an Stabilisatoren im Falle der Peressigsäure völlig ungenügend ist. Die Lösungen der Beispiele 1 und 4 waren leicht getrübt, jene der Beispiele 2, 3 und 5 völlig klar.

**Vergleichsbeispiel 3**

Hergestellt wurde eine 5 gew.-%ige Gleichgewichtsperessigsäure unter Verwendung der gleichen Menge 50 gew.-%iger wäßriger $H_2O_2$-Lösung, Essigsäure und $H_2SO_4$ wie in den Beispielen 1 bis 5 und Vergleichsbeispielen 1 und 2.

Die eingesetzte $H_2O_2$-Lösung (470 g) wurde zwei Tage vor der Umsetzung zu Peressigsäure mit einer Natriumstannat ($Na_2SnO_3 \cdot 3 H_2O$) und HEDP (1-Hydroxyethan-1,1-diphosphonsäure) als einzige Stabilisatoren enthaltenden konzentrierten wäßrigen Stabilisatorlösung in einer solchen Menge versetzt, daß sie 30 mg $Na_2SnO_3 \cdot 3 H_2O$ und 16,8 mg HEDP enthielt. Aus der so stabilisierten $H_2O_2$-Lösung wurde zwei Tage später die Peressigsäure hergestellt, wobei kein weiterer Stabilisator zugesetzt wurde. Die Peressigsäurelösung enthielt somit 50 ppm $Na_2SnO_3 \cdot 3 H_2O$ und 28 ppm HEDP. Diese Menge entsprach im wesentlichen derjenigen des Beispiels 2.

Bei der adiabatischen Warmlagerung der gemäß Vergleichsbeispiel 3 hergestellten Gleichgewichtsperessigsäure wird der SADT-Test "mindestens 60 °C" nicht erfüllt.

**Patentansprüche**

1. Verfahren zur Herstellung von Percarbonsäurelösungen, welche den SADT-Test "mindestens 60 °C" erfüllen, durch Umsetzung der Carbonsäure mit wäßrigem Wasserstoffperoxid in Gegenwart saurer Katalysatoren und, sofern erwünscht, Überführung der im Reaktionsgemisch enthaltenden Percarbonsäure in andere Lösungsmittel,
dadurch gekennzeichnet,
daß man mindestens eine Zinnverbindung in einer Menge von mindestens 20 ppm, berechnet als Sn und bezogen auf die Percarbonsäurelösung, unmittelbar vor der Umsetzung einem Reaktionspartner oder während und/oder nach der Umsetzung dem Reaktionsgemisch als Stabilisator zusetzt.

2. Verfahren nach Anspruch 1,
dadurch gekennzeichnet,
daß man die Zinnverbindung in einer Menge von 20 bis 200 ppm, insbesondere 20 bis 50 ppm, berechnet als Sn und bezogen auf die Percarbonsäurelösung, zusetzt.

3. Verfahren nach Anspruch 1 oder 2,
dadurch gekennzeichnet,
daß man als Carbonsäure Essigsäure oder Propionsäure verwendet und die Umsetzung in Gegenwart einer Mineralsäure, insbesondere Schwefelsäure, bis zur Gleichgewichtseinstellung durchführt.

4. Verfahren nach einem der Ansprüche 1 bis 3,
dadurch gekennzeichnet,
daß man zusätzlich einen oder mehrere Stabilisatoren aus der Reihe der (a) Chelatkomplexbildner auf der Basis von Phosphonsäuren, insbesondere Hydroxy- und Aminophosphonsäuren, Aminocarbonsäuren, Hydroxycarbonsäuren, N-heterocyclischen Carbonsäuren sowie Salzen der genannten Säuren, (b) Pyro- und Polyphosphorsäure sowie Salzen der genannten Säuren und (c) Radikalfänger auf der Basis alkylierter Hydroxyaromaten, einsetzt.

5. Verfahren nach einem oder mehreren der Ansprüche 1 bis 4,
dadurch gekennzeichnet,
daß man als Stabilisator eine anorganische Zinn(IV)verbindung, vorzugsweise ein Stannat und insbesondere $Na_2SnO_3 \cdot 3H_2O$, in einer Menge von 20 bis 200 ppm, berechnet als Sn, verwendet und zusätzlich mindestens einen weiteren Stabilisator aus der Reihe der Hydroxyphosphonsäuren, Aminophosphonsäuren, Aminocarbonsäuren, Hydroxycarbonsäuren, N-heterocyclischen Carbonsäuren, Pyro- und Polyphosphorsäure oder Salzen der genannten Säuren in einer Menge von 20 bis 1000 ppm, bezogen auf die Percarbonsäurelösung, einsetzt.

6. Verfahren nach einem oder mehreren der Ansprüche 1 bis 5,
dadurch gekennzeichnet,
daß man aus einer anorganischen Zinnverbindung und mindestens einem Chelatkomplexbildner aus der Reihe der Hydroxy- und Aminophosphonsäuren, eine klare wäßrige Stabilisatorlösung herstellt und diese während und/oder nach der Umsetzung dem Reaktionsgemisch in einer solchen Menge zusetzt, daß in der gebildeten Percarbonsäurelösung die Sn-Konzentration insgesamt 20 bis 200 ppm, vorzugsweise 20 bis 50 ppm, und die Konzentration an Phosphonsäurestabilisatoren insgesamt 20 bis 200 ppm beträgt.

7. Stabilisierte Percarbonsäurelösungen, welche den SADT-Test "mindestens 60 °C" erfüllen, enthaltend eine Percarbonsäure, ein oder mehrere Lösungsmittel und mindestens einen Stabilisator,
dadurch gekennzeichnet,
daß sie als Stabilisator eine Zinnverbindung in einer Menge von mindestens 20 ppm, berechnet als Sn, enthalten, erhältlich nach einem Verfahren der Ansprüche 1 bis 6.

8. Stabilisierte Percarbonsäurelösungen nach Anspruch 7,
dadurch gekennzeichnet,
daß sie als Percarbonsäure eine wasserlösliche Percarbonsäure, vorzugsweise Peressigsäure oder Perpropionsäure und als Lösungsmittel die der Percarbonsäure zugrundeliegende Carbonsäure und Wasser und zusätzlich Wasserstoffperoxid und Mineralsäure enthalten.

9. Stabilisierte Percarbonsäurelösungen nach Anspruch 8,
dadurch gekennzeichnet,
daß es sich um sogenannte Gleichgewichtsperessigsäure handelt, welche einen Gehalt von
2,5 bis 40 Gew.-% Peressigsäure,
5 bis 74 Gew.-% Essigsäure,
1 bis 30 Gew.-% Wasserstoffperoxid,
10 bis 60 Gew.-% Wasser,
0,05 bis max. 1 Gew.-% Mineralsäure, insbesondere $H_2SO_4$, und vorzugsweise einen Gehalt von

4 bis 20 Gew.-% Peressigsäure,

5 bis 74 Gew.-% Essigsäure,

1 bis 30 Gew.-% Wasserstoffperoxid,

15 bis 60 Gew.-% Wasser,

0,05 bis max. 1 Gew.-% Schwefelsäure

aufweisen und als Stabilisator eine anorganische Zinnverbindung, vorzugsweise ein Stannat der Formel Me$_2$SnO$_3$ • 3H$_2$O, worin Me für ein Alkalimetall oder Ammonium steht, in einer Menge von 20 bis 200 ppm, vorzugsweise 20 bis 50 ppm, jeweils berechnet als Sn, enthalten.

**10.** Stabilisierte Percarbonsäurelösungen nach Anspruch 9,
dadurch gekennzeichnet,
daß sie zusätzlich einen Chelatkomplexbildner auf der Basis einer 1-Hydroxyalkyliden-1,1-diphosphonsäure oder Aminophosphonsäure in einer Menge von 20 bis 1000 ppm, vorzugsweise 20 bis 200 ppm, enthalten.

**Claims**

1. Method for the preparation of solutions of percarboxylic acids which meet the SADT Test "at least 60°C" by reacting the carboxylic acid with aqueous hydrogen peroxide in the presence of acid catalysts and, optionally, transferring the percarboxylic acid contained in the reaction mixture into other solvents, characterised in that at least one tin compound, in a quantity of at least 20 ppm, calculated as Sn and with reference to the solution of percarboxylic acid, is added as a stabiliser to one co-reactant immediately prior to the reaction or to the reaction mixture during and/or after the reaction.

2. Method according to claim 1, characterised in that the tin compound is added in a quantity of from 20 to 200 ppm, particularly from 20 to 50 ppm, calculated as Sn and with reference to the solution of percarboxylic acid.

3. Method according to claims 1 or 2, characterised in that the carboxylic acid used is acetic acid or propionic acid and the reaction is carried out in the presence of a mineral acid, in particular sulphuric acid, until equilibrium is established.

4. Method according to one of claims 1 to 3, characterised in that in addition one or more stabilisers selected from (a) the chelating agents based on phosphonic acids, in particular hydroxyphosphonic acids and aminophosphonic acids, aminocarboxylic acids, hydroxycarboxylic acids, N-heterocyclic carboxylic acids, and salts of the said acids, (b) pyrophosphoric and polyphosphoric acid and salts of the said acids and (c) free-radical scavengers based on alkylated hydroxyaromatic compounds are used.

5. Method according to one or more of claims 1 to 4, characterised in that the stabiliser used is an inorganic tin(IV) compound, preferably a stannate and in particular Na$_2$SnO$_3$ . 3H$_2$O, in a quantity of from 20 to 200 ppm, calculated as Sn, and in addition at least one other stabiliser selected from hydroxyphosphonic acids, aminophosphonic acids, aminocarboxylic acids, hydroxycarboxylic acids, N-heterocyclic carboxylic acids, pyrophosphoric acid and polyphosphonic acid, or salts of the said acids is used, in a quantity of from 20 to 1000 ppm, with reference to the solution of percarboxylic acid.

6. Method according to one or more of claims 1 to 5, characterised in that a clear aqueous stabilising solution is prepared from an inorganic tin compound and at least one chelating agent selected from hydroxyphosphonic acids and aminophosphonic acids, and the said solution is added to the reaction mixture during and/or after the reaction, in a quantity such that in the solution of percarboxylic acid formed the Sn concentration is in total from 20 to 200 ppm, preferably from 20 to 50 ppm, and the concentration of phosphonic acid stabilisers is from 20 to 200 ppm in total.

7. Stabilised solutions of percarboxylic acids, which meet the SADT Test "at least 60°C", containing a percarboxylic acid, one or more solvents and at least one stabiliser, characterised in that they contain as stabiliser a tin compound in a quantity of at least 20 ppm, calculated as Sn, obtainable by a method according to claims 1 to 6.

EP 0 563 584 B1

8. Stabilised solutions of percarboxylic acids according to claim 7, characterised in that they contain as percarboxylic acid a water-soluble percarboxylic acid, preferably peracetic acid or perpropionic acid, and as solvent the carboxylic acid corresponding to the percarboxylic acid and water and additionally hydrogen peroxide and mineral acids.

9. Stabilised solutions of percarboxylic acids according to claim 8, characterised in that they are a so-called equilibrium peracetic acid having a content of
from 2.5 to 40% by weight of peracetic acid,
from 5 to 74% by weight of acetic acid,
from 1 to 30% by weight of hydrogen peroxide,
from 10 to 60% by weight of water,
from 0.05 up to a maximum of 1% by weight of mineral acid, in particular $H_2SO_4$, and preferably having a content of
from 4 to 20% by weight of peracetic acid,
from 5 to 74% by weight of acetic acid,
from 1 to 30% by weight of hydrogen peroxide,
from 15 to 60% by weight of water,
from 0.05 up to a maximum of 1% by weight of sulphuric acid and the stabiliser they contain is an inorganic tin compound, preferably a stannate of the formula $Me_2SnO_3 . 3H_2O$, wherein Me represents an alkali metal or ammonium, in a quantity of from 20 to 200 ppm, preferably from 20 to 50 ppm, in each case calculated as Sn.

10. Stabilised solutions of percarboxylic acids according to claim 9, characterised in that they contain in addition a chelating agent based on a 1-hydroxyalkylidene-1,1-diphosphonic acid or aminophosphonic acid in a quantity of from 20 to 1000 ppm, preferably of from 20 to 200 ppm.

**Revendications**

1. Procédé pour la fabrication de solutions d'acides percarboxyliques qui satisfont au test SADT "au moins 60 °C" par réaction entre l'acide carboxylique et du peroxyde d'hydrogène aqueux en présence de catalyseurs acides et, le cas échéant, en faisant passer l'acide percarboxylique contenu dans le mélange réactionnel dans d'autres solvants caractérisé en ce qu'on ajoute au mélange réactionnel comme stabilisateur au moins un composé de l'étain en une quantité d'au moins 20 ppm, calculée sous forme de Sn et par rapport à la solution d'acide percarboxylique directement avant la transformation d'un partenaire de réaction ou pendant et/ou après la réaction.

2. Procédé selon la revendication 1, caractérisé en ce qu'on ajoute le composé de l'étain en une quantité comprise entre 20 et 200 ppm, en particulier entre 20 et 50 ppm, calculée sous forme de Sn et par rapport à la solution d'acide percarboxylique.

3. Procédé selon la revendication 1 ou 2, caractérisé en ce qu'on utilise comme acide carboxylique de l'acide acétique ou de l'acide propionique et en ce qu'on réalise la réaction en présence d'un acide minéral, en particulier d'acide sulfurique, jusqu'à l'établissement de l'équilibre.

4. Procédé selon une quelconque des revendications 1 à 3, caractérisé en ce qu'on ajoute en outre un ou plusieurs stabilisateurs de la série (a) des agents de formation de complexes chélatés à base d'acides phosphoniques, en particulier d'acides hydroxyphosphoniques et aminophosphoniques, d'acides aminocarboxyliques, d'acides hydroxycarboxyliques, d'acides carboxyliques N-hétérocycliques ainsi que de sels des acides mentionnés, (b) des acides pyrophosphoriques et polyphosphoriques ainsi que des sels des acides mentionnés et (c) des capteurs de radicaux à base d'hydroxyaromates alkylés.

5. Procédé selon une ou plusieurs des revendications 1 à 4, caractérisé en ce qu'on utilise comme stabilisateur un composé inorganique de l'étain (IV), de préférence un stannate et en particulier du $Na_2SnO_3.3H_2O$, en une quantité comprise entre 20 et 200 ppm, calculée sous forme de Sn, et en ce qu'on utilise au moins un autre stabilisateur de la série des acides hydroxyphosphoniques, aminophosphoniques, aminocarboxyliques, hydroxycarboxyliques, carboxyliques N-hétérocycliques, pyrophosphoriques et polyphosphoriques ou des sels des acides mentionnés en une quantité comprise entre 20 et 1000 ppm, par rapport à la solution d'acide percarboxylique.

9

**6.** Procédé selon une ou plusieurs des revendications 1 à 5, caractérisé en ce qu'on prépare une solution aqueuse claire de stabilisateur à partir d'un composé inorganique de l'étain et d'au moins un agent de formation de complexes chélatés de la série des acides hydroxyphosphoniques et aminophosphoniques et en ce qu'on ajoute celle-ci pendant et/ou après la réaction du mélange réactionnel en une quantité telle que la concentration en Sn dans la solution d'acide percarboxylique formée est comprise au total entre 20 et 200 ppm, de préférence entre 20 et 50 ppm, et que la concentration en stabilisateurs d'acide phosphonique est comprise au total entre 20 et 200 ppm.

**7.** Solutions stabilisées d'acides percarboxyliques qui satisfont au test SADT "au moins 60 °C", contenant un acide percarboxylique, un ou plusieurs solvants et au moins un stabilisateur, caractérisées en ce qu'elles contiennent comme stabilisateur un composé de l'étain en une quantité d'au moins 20 ppm, calculée sous forme de Sn, et en ce qu'elles sont obtenues selon un procédé des revendications 1 à 6.

**8.** Solutions stabilisées d'acides percarboxyliques selon la revendication 7, caractérisées en ce qu'elles contiennent comme acide percarboxylique un acide percarboxylique soluble dans l'eau, de préférence de l'acide peracétique ou de l'acide perpropionique, et comme solvant l'acide carboxylique qui est à la base de l'acide percarboxylique et de l'eau ainsi que du peroxyde d'hydrogène et un acide minéral.

**9.** Solutions stabilisées d'acides percarboxyliques selon la revendication 8, caractérisées en ce qu'il s'agit de ce qu'on appelle de l'acide peracétique à l'équilibre, présentant les teneurs suivantes:
2,5 à 40 % en poids d'acide peracétique
5 à 74 % en poids d'acide acétique
1 à 30 % en poids de peroxyde d'hydrogène
10 à 60 % en poids d'eau
0,05 à max. 1 % en poids d'un acide minéral, en particulier du $H_2SO_4$,
et de préférence les teneurs suivantes:
4 à 20 % en poids d'acide peracétique
5 à 74 % en poids d'acide acétique
1 à 30 % en poids de peroxyde d'hydrogène
15 à 60 % en poids d'eau
0,05 à max. 1 % en poids de $H_2SO_4$,
et contenant comme stabilisateur un composé inorganique de l'étain, de préférence un stannate de formule $Me_2SnO_3.3H_2O$, Me représentant un métal alcalin ou de l'ammonium, en une quantité comprise entre 20 et 200 ppm, de préférence entre 20 et 50 ppm, calculée sous forme de Sn.

**10.** Solutions stabilisées d'acides percarboxyliques selon la revendication 9, caractérisées en ce qu'elles contiennent en outre un agent de formation de complexes chélatés à base d'un acide 1-hydroxyalkylidène-1,1-diphosphonique ou d'un acide aminophosphonique en une quantité comprise entre 20 et 1000 ppm, de préférence entre 20 et 200 ppm.